# EUROPÄISCHE PATENTSCHRIFT

(11) **EP 1 019 032 B1**
(45) Veröffentlichungstag und Bekanntmachung des Hinweises auf die Patenterteilung: **27.11.2002**
(21) Anmeldenummer: 98950053.3
(22) Anmeldetag: 18.09.1998
(51) Int. Cl.: A61K 9/70

(54) **VERFAHREN ZUR VERMEIDUNG DES MISSBRAUCHS EINES TRANSDERMALEN THERAPEUTISCHEN SYSTEMS**
METHOD FOR PREVENTING THE MISUSE OF A TRANSDERMAL THERAPEUTIC SYSTEM
PROCEDE POUR EVITER LE MAUVAIS USAGE D'UN SYSTEME THERAPEUTIQUE TRANSDERMIQUE

(30) Priorität: 01.10.1997 DE 19743484
(43) Veröffentlichungstag der Anmeldung: 19.07.2000
(73) Patentinhaber: LTS Lohmann Therapie-Systeme AG, 56626 Andernach (DE)
(72) Erfinder: BECHER, Frank, D-56072 Koblenz (DE); KLINK, Ann-Katrin, D-56323 Waldesch (DE)
(74) Vertreter: Flaccus, Rolf-Dieter, Dr.
(86) Internationale Anmeldenummer: EP9805955
(87) Internationale Veröffentlichungsnummer: WO99016428

(56) Entgegenhaltungen:
- WO-A-89/07959
- WO-A-90/04965
- US-A- 5 149 538

## Beschreibung

Die Erfindung betrifft ein Verfahren zum Schutz einer Person vor Kontamination infolge unzulässiger oraler Applikation toxischer Inhaltsstoffe eines transdermalen therapeutischen Systems (TTS), umfassend zumindest eine wirkstoffhaltige, insbesondere haftklebend ausgerüstete Schicht, sowie wenigstens eine weitere, für den Wirkstoff undurchlässige Rückschicht, sowie ein TTS mit einem Vergällungsstoff.

Bei einer Reihe von TTS besteht die akute Gefahr, daß unerwünschte Auswirkungen eintreten können, wenn sich Personen - sei es aus neuen oder aus abgenommenen Systemen - oral einen daraus lösbaren Wirkstoff zuführen. Beispielsweise haben Kleinkinder die Eigenschaft, alles Interessante in den Mund zu stecken und zumindest daran zu nuckeln oder zu kauen. Dies kann besonders dann nicht verhindert werden, wenn Kinder zufälligerweise Zugriff zu derartigen Systemen, insbesondere ohne Release Liner, erlangen können.
Bisher sind derartige Zwischenfälle zwar nicht bekannt geworden, aber sie werden von den Arzneimittelbehörden mehr und mehr befürchtet, die aus diesem Grunde auch verschiedentlich die Forderung nach kindersicheren Verpackungen gestellt haben. Tatsächlich sind solche Verpackungen in verschiedentlicher Ausführung entwickelt worden. Sie schützen aber nicht davor, daß ein Kind ein TTS in den Mund nimmt, wenn die Packung einmal geöffnet worden ist, oder wenn ein benutztes Pflaster irgendwie in ihre Hände gerät. Besonders bei TTS mit Wirkstoffen wie Betäubungsmitteln, schmerzlindernden Medikamenten, Tranquilizern oder Psychopharmaka könnte ein oraler Mißbrauch zu schweren Gesundheitsschädigungen führen. Andererseits könnten auch Süchtige in Versuchung geraten, solche Wirkstoffe durch Lutschen oder Kauen aus transdermalen therapeutischen Systemen herauszulösen.

Es ist bereits bekannt, trinkbaren Ethylalkohol zu vergällen und damit als Brennspiritus in den Handel zu bringen. Eine Vergällung von Arzneimittel-Wirkstoffen ist dagegen bisher nicht bekannt geworden.

Der Erfindung liegt die Aufgabe zugrunde, ein Verfahren zum Schutz einer Person der im Oberbegriff von Anspruch 1 genannten Art so auszugestalten, daß ein oraler Mißbrauch der vorgängig geschilderten Art nicht möglich ist, wobei andererseits aber eine nachteilige Veränderung des therapeutischen Wirkstoffs im System vermieden und ebenfalls eine Gesundheitsschädigung bei der unzulässigen oralen Applikation ausgeschlossen sein soll.

Zur Lösung der Aufgabe wird mit dem erfindungsgemäßen Verfahren vorgeschlagen, daß ein therapeutisch neutraler und nicht interagierender Stoff von widerwärtigem Geschmack beigemischt wird.
Weil dieser Stoff höchst unangenehme und unerwartete Geschmackserlebnisse hervorruft, genügt ein erster oraler Kontakt mit dem erfindungsgemäßen TTS oder einer seiner vergällten Schichten zu einer spontanen Reaktion, um das entsprechende Pflaster sofort auszuspucken und damit Kinder oder Drogensüchtige von der unzulässigen oralen Applikation des im Pflaster enthaltenen Wirkstoffs abzuhalten.

Ein Ausgestaltung sieht vor, daß zur Vergällung eines TTS ein Stoff verwendet wird, welcher Brechreiz verursacht und damit dem Betroffenen die Versuchung zur oralen Applikation verleidet.

Eine Ausgestaltung des Verfahrens sieht erfindungsgemäß vor, daß der Stoff in einer separaten, extrem dünnen und die Permeation des Wirkstoffs nicht behindernde Schicht auf die wirkstoffhaltige Schicht aufgetragen wird.
Dies hat den Vorteil, daß schon bei der ersten Berührung der Mundschleimhaut oder der Zunge mit dem TTS noch vor Kontaminierung mit der eigentlichen wirkstoffhaltigen Schicht ein äußerst widerwärtiges Geschmackserlebnis die weitere orale Kontaktaufnahme verleidet.

Dabei kann weiter von der Maßnahme Gebrauch gemacht sein, daß der Stoff in einer separaten, vorzugsweise sehr dünnen Schicht zusätzlich auf die Rückschicht aufgetragen wird. Damit wird eine weitere Intensivierung des Vergällungs-Effektes sicher erreicht.

Es ist dabei vorgesehen, daß die separate Schicht des Stoffes in einer Dicke zwischen 10 und 100 µm, bevorzugt zwischen 5 und 20 µm aufgetragen wird.

Eine besonders bevorzugte Ausgestaltung der Erfindung sieht weiter vor, daß ein Stoff verwendet wird, der eine Reizung wie Brennen der Schleimhäute der Mundhöhle und der Zunge verursacht. Eine einmal insoweit erlebte negative Erfahrung dürfte genügen, ein Kind oder einen Süchtigen von weiteren Versuchen einer oralen Kontaktnahme mit einem TTS dauerhaft abzuhelfen. Im übrigen kann eine ähnliche Wirkung auch erzielt werden, wenn ein Stoff verwendet wird, der einen intensiv bitteren Geschmack und insbesondere Nachgeschmack verursacht.

Ein wirkungsvolle Ausgestaltung des Verfahrens nach der Erfindung sieht vor, daß zu den Naturierung bzw. Vergällung wenigstens einer Schicht eines TTS als Bitterstoffes bzw. widerwärtig schmeckende Substanzen wie Gallsäure, Chinin, Tannin, Angostura, Coffein (pur), Lobelin, Teebaumöl, gewisse Schimmelpilzkulturen, vergällte oder geronnene Substanzen, Terpentin oder Ammoniak verwendet werden.

Zur Durchführung einer Ausführungsform des Verfahrens ist vorgesehen, daß die zur Vergällung verwendeten Stoffe in eine Überzugsschicht eingearbeitet werden, mit welcher die wirkstoffhaltige Schicht und fallweise auch die Rückschicht überzogen werden. Dabei ist als besonders bevorzugte Lösung vorgesehen, daß eine Überzugsschicht verwendet wird, die in der Mundflüssigkeit löslich ist.

Das Verfahren ist unkompliziert und wirksam und schützt Kinder und/oder Süchtige vor unzulässiger oraler Applikation toxischer Inhaltsstoffe eines transdermalen therapeutischen Systems. Damit lösen das Verfahren und das erfindungsgemäße TTS in optimaler Weise die eingangs gestellte Aufgabe.

## Patentansprüche

1. Transdermales therapeutisches System (TTS), umfassend zumindest eine wirkstoffhaltige, insbesondere haftklebend ausgerüstete Schicht, sowie wenigstens eine weitere, für den Wirkstoff undurchlässige Rückschicht, **dadurch gekennzeichnet, daß** es einen therapeutisch neutralen und nicht mit dem Wirkstoff interagierenden Stoff von widerwärtigem Geschmack enthält.

2. TTS nach Anspruch 1, **dadurch gekennzeichnet, daß** der Stoff mit widerwärtigem Geschmack zumindest in der wirkstoffhaltigen Schicht enthalten ist.

3. TTS nach Anspruch 1 oder 2, **dadurch gekennzeichnet, daß** die zur Vergällung verwendeten Stoffe in eine Überzugsschicht eingearbeitet sind, mit welcher die wirkstoffhaltige und fallweise auch die Rückschicht überzogen sind.

4. TTS nach Anspruch 3, **dadurch gekennzeichnet, daß** die Überzugsschicht in der Mundflüssigkeit löslich ist.

5. TTS nach einem oder mehreren der Ansprüche 1 bis 4, **dadurch gekennzeichnet, daß** der genannte Stoff ein Brechreiz verursachender Stoff ist.

6. TTS nach einem oder mehreren der Ansprüche 1 bis 5, **dadurch gekennzeichnet, daß** der genannte Stoff in einer separaten, extrem dünnen und die Permeation des Wirkstoffes nicht behindernden Schicht auf die wirkstoffhaltige Schicht aufgetragen ist.

7. TTS nach einem oder mehreren der Ansprüche 1 bis 6, **dadurch gekennzeichnet, daß** der genannte Stoff in einer separaten, extrem dünnen Schicht zusätzlich auf der Rückschicht aufgetragen ist.

8. TTS nach einem oder mehreren der Ansprüche 1 bis 7, **dadurch gekennzeichnet, daß** die separate Schicht des Stoffes in einer Dicke zwischen 10 und 100 µm und bevorzugt zwischen 5 und 20 µm aufgetragen ist.

9. TTS nach einem oder mehreren der Ansprüche 1 bis 8, **dadurch gekennzeichnet, daß** der Stoff von widerwärtigem Geschmack ein Stoff ist, der eine Reizung wie Brennen der Schleimhäute der Mundhöhle und der Zunge verursacht.

10. TTS nach einem oder mehreren der Ansprüche 1 bis 9, **dadurch gekennzeichnet, daß** der Stoff von widerwärtigem Geschmack ein Stoff ist, der einen intensiv bitteren Geschmack und insbesondere Nachgeschmack verursacht.

11. TTS nach einem oder mehreren der Ansprüche 1 bis 10, **dadurch gekennzeichnet, daß** wenigstens eine Schicht des TTS zur Denaturierung bzw. Vergällung Gallsäure, Chinin, Tannin, Angostura, Coffein (pur), Lobelin, Teebaumöl, Schimmelpilzkulturen, vergällte oder geronnene Substanzen, Terpentin oder Ammoniak als Bitterstoffe bzw. widerwärtig schmeckende Substanzen enthält.

12. Verwendung eines therapeutisch neutralen Stoffes von widerwärtigem Geschmack zur Herstellung eines transdermalen therapeutischen Systems, welches dadurch gegen unzulässige orale Applikation geschützt ist, wobei das System zumindest eine wirkstoffhaltige, insbesondere haftklebend ausgerüstete Schicht, sowie wenigstens eine weitere, für den Wirkstoff undurchlässige Rückschicht umfaßt, und wobei der genannte Stoff nicht mit dem Wirkstoff interagiert.

## Claims

1. Transdermal therapeutic system (TTS) comprising at least one active substance-containing layer, more particularly an active substance-containing layer which has been rendered pressure-sensitive adhesive, as well as at least one further backing layer impermeable to the active substance, **characterized in that** it contains a substance which is therapeutically neutral and does not interact with the active substance and which has a repugnant taste.

2. TTS according to claim 1, **characterized in that** the substance of repugnant taste is contained at least in the active substance-containing layer.

3. TTS according to claim 1 or 2, **characterized in that** the substances used for denaturing are incorporated in a coat layer with which the active substance-containing layer and, as the case may be, the cover layer are coated.

4. TTS according to claim 3, **characterized in that** the coating layer is soluble in the oral fluid.

5. TTS according to one or more of claims 1 to 4, **characterized in that** the said substance is a substance causing nausea.

6. TTS according to one or more of claims 1 to 5, **characterized in that** the said substance is applied to the active substance-containing layer in a separate, extremely thin layer not impairing the permeation of the active substance.

7. TTS according to one or more of claims 1 to 6, **characterized in that** the said substance is additionally applied to the backing layer, in a separate, extremely thin layer.

8. TTS according to one or more of claims 1 to 7, **characterized in that** the separate layer of the said substance is applied in a thickness of between 10 and 100 µm, and preferably in a thickness of between 5 and 20 µm.

9. TTS according to one or more of claims 1 to 8, **characterized in that** the substance of repugnant taste is a substance causing an irritation such as burning of the mucosae of the oral cavity and the tongue.

10. TTS according to one or more of claims 1 to 9, **characterized in that** the substance of repugnant taste is a substance causing an intensely bitter taste and, in particular, aftertaste.

11. TTS according to one or more of claims 1 to 10, **characterized in that**, for denaturation or producing a repugnant taste, at least one layer of the TTS contains gallic acid, quinine, tannin, angostura, caffeine (pure), lobeline, tea tree oil, mould cultures, denatured or coagulated substances, turpentine or ammonia as amaroids or, respectively, substances having a repugnant taste.

12. Use of a therapeutically neutral substance of repugnant taste for preparing a transdermal therapeutic system which is thereby protected against impermissible oral application, said system comprising at least one active substance-containing layer, more particularly an active substance-containing layer which has been rendered pressure-sensitive adhesive, as well as at least one further backing layer impermeable to the active substance, and with said substance not interacting with the active substance.

## Revendications

1. Système thérapeutique transdermique (STT), comprenant au moins une couche contenant au moins une substance active, en particulier équipée de manière adhésive, ainsi qu'au moins une autre couche de base imperméable à la substance active, **caractérisé en ce qu'**il contient une substance thérapeutiquement neutre et n'interagissant pas avec la substance active d'un goût désagréable.

2. STT selon la revendication 1, **caractérisé en ce que** la substance d'un goût désagréable est au moins contenue dans la couche contenant la substance active.

3. STT selon la revendication 1 ou 2, **caractérisé en ce que** les substances dénaturantes sont incorporées dans une couche de revêtement par laquelle la couche contenant la substance active et éventuellement aussi la couche de base sont revêtues.

4. STT selon la revendication 3, **caractérisé en ce que** la couche de revêtement est soluble dans la salive.

5. STT selon l'une ou plusieurs des revendications 1 à 4, **caractérisé en ce que** la substance mentionnée est une substance provoquant des nausées.

6. STT selon l'une ou plusieurs des revendications 1 à 5, **caractérisé en ce que** la substance mentionnée est appliquée sur la couche contenant la substance active, dans une couche séparée, extrêmement mince et ne gênant pas la perméation de la substance active.

7. STT selon l'une ou plusieurs des revendications 1 à 6, **caractérisé en ce que** la substance mentionnée est appliquée en supplément dans une couche séparée, extrêmement mince sur la couche de base.

8. STT selon l'une ou plusieurs des revendications 1 à 7, **caractérisé en ce que** la couche séparée de la substance est appliquée en une épaisseur entre 10 et 100 µm et de préférence entre 5 et 20 µm.

9. STT selon l'une ou plusieurs des revendications 1 à 8, **caractérisé en ce que** la substance d'un goût désagréable est une substance qui provoque une irritation, telle qu'une brûlure de la muqueuse de la cavité buccale et de la langue.

10. STT selon l'une ou plusieurs des revendications 1 à 9, **caractérisé en ce que** la substance d'un goût désagréable est une substance qui provoque un goût et en particulier un arrière-goût amer intense.

11. STT selon l'une ou plusieurs des revendications 1 à 10, **caractérisé en ce qu'**au moins une couche du STT contient, pour la dénaturation , de l'acide gallique, de la quinine, du tanin, de l'angostura, de la caféine (pure), de la lobéline, de l'huile d'arbre à thé, des cultures de moisissures, des substances dénaturées ou coagulées, de la térébenthine ou de l'ammoniaque comme substances amères ou substances d'un goût désagréable.

12. Utilisation d'une substance thérapeutiquement neutre d'un goût désagréable pour la préparation d'un système thérapeutique transdermique qui est protégé contre une administration orale non autorisée, le système comprenant au moins une couche contenant une substance active, en particulier équipée de manière adhésive, ainsi qu'au moins une autre couche de base imperméable à la substance active et la substance mentionnée n'interagissant pas avec la substance active.
